# EUROPEAN PATENT APPLICATION

(11) **EP 3 505 047 A1**
(43) Date of publication of application: **03.07.2019**
(21) Application number: 17210613.0
(22) Date of filing: 27.12.2017
(51) Int. Cl.: A61B 5/00

(54) **DEVICE FOR IMAGING SKIN**

(71) Applicant: Koninklijke Philips N.V., 5656 AE Eindhoven (NL)
(72) Inventor: RAS, Arnoldus Johannes Martinus Jozeph, 5656 AE Eindhoven (NL); HERMANS, Walter, 5656 AE Eindhoven (NL); VARGHESE, Babu, 5656 AE Eindhoven (NL)
(74) Representative: de Haan, Poul Erik

(57) **Abstract**

There is provided a device (100) for imaging skin. The device (100) comprises a light source (104) configured to emit light to illuminate the skin (102). The light source (104) has a radiation angle in a range from 5 to 40 degrees. The radiation angle is the angle at which an intensity of the light emitted by the light source (104) is half that of a maximum intensity of the light emitted by the light source (104). The device (100) also comprises an imaging sensor (108) configured to detect light reflected from the skin (102) and convert the detected light into an image of the skin (102).

## Description

### FIELD OF THE INVENTION

The invention relates to a device for imaging skin, a method of operating the device to image skin and a system for measuring skin texture.

### BACKGROUND OF THE INVENTION

In the skincare field, it is valuable to acquire images of the skin. For example, images of the skin can be useful for monitoring the state of the skin (such as any deterioration or improvement in the state of the skin) and to detect any skin conditions.

Some existing devices for imaging skin comprise a light source for illuminating the skin while an image is acquired by an imaging sensor of the device. JP 2013/026846 discloses an example of a skin observation device where light is applied to the skin. This skin observation device uses a different angle of incidence of light with respect to the optical axis for observing skin texture to that used for observing skin spots.

However, while there exist such devices for imaging skin, the existing devices can suffer from poor image quality. For example, the images acquired by the existing devices are often not of a high enough resolution for features of the skin (such as skin texture, fine lines and wrinkles, skin spots, impurities, irregularities, abnormalities, etc.) to be resolved to an adequate degree.

There is thus a need for an improved device for imaging skin, which overcomes the existing problems.

### SUMMARY OF THE INVENTION

As noted above, a limitation with existing devices is that the images acquired by such devices are often not of a high enough resolution for features of the skin (such as skin texture, fine lines and wrinkles, skin spots, impurities, irregularities, abnormalities, etc.) to be adequately resolved. It would thus be valuable to have an improved device for imaging skin, which overcomes the existing problems.

Therefore, according to a first aspect, there is provided a device comprising a light source configured to emit light to illuminate the skin. The light source has a radiation angle in a range from 5 to 40 degrees. The radiation angle is the angle at which an intensity of the light emitted by the light source is half that of a maximum intensity of the light emitted by the light source. The device also comprises an imaging sensor configured to detect light reflected from the skin and convert the detected light into an image of the skin.

In some embodiments, the light source may have a radiation angle in a range from 10 to 30 degrees. In some embodiments, the light source may have a radiation angle in a range from 10 to 25 degrees.

In some embodiments, the light source may be configured to emit light with an angle of incidence in a range from 5 to 50 degrees. In some embodiments, the light source may be configured to emit light with an angle of incidence in a range from 10 to 30 degrees. In some embodiments, the light source may be configured to emit light with an angle of incidence in a range from 10 to 25 degrees.

In some embodiments, the light source may be configured to emit light to uniformly illuminate the skin. In some embodiments, the light source may comprise a plurality of light emitters. In some embodiments, the light source may comprise at least three light emitters.

In some embodiments, the device may further comprise a housing configured to house the light source and the imaging sensor. In some embodiments, the housing may comprise an aperture through which the light source is configured to emit light and the imaging sensor is configured to detect light reflected from the skin.

In some embodiments, the aperture may be a numerical aperture. In some embodiments, the numerical aperture may have a value in a range from 0.005 to 0.3.

In some embodiments, a distance between the light source and the imaging sensor may be greater than 5 millimeters.

According to a second aspect, there is provided a system for measuring skin texture. The system comprises a device as described above and a processor configured to acquire the image of the skin from the imaging sensor and process the acquired image to measure the skin texture.

According to a third aspect, there is provided a method of operating a device to image skin. The device comprises a light source and an imaging sensor. The method comprises emitting light from the light source to illuminate the skin. The light source has a radiation angle in a range from 5 to 40 degrees. The radiation angle is the angle at which an intensity of the light emitted by the light source is half that of a maximum intensity of the light emitted by the light source. The method also comprises detecting, by the imaging sensor, light reflected from the skin and converting, by the imaging sensor, the detected light into an image of the skin.

According to a fourth aspect, there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitations of existing devices are addressed. In particular, according to the above-described aspects and embodiments, images of improved resolution (and thus improved quality) can be acquired. The resolution of the images that is achieved is optimum for features of the skin (such as skin texture, fine lines and wrinkles, skin spots, impurities, irregularities, abnormalities, etc.) to be adequately resolved in the images. In this way, the images can be used to provide more accurate skin monitoring and to more reliably detect skin conditions. There is thus provided an improved device for imaging skin, which overcomes existing problems.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic diagram of a device according to an embodiment;
Fig. 2 is a schematic diagram of a radiation angle range for a light source of a device according to an embodiment;
Figs. 3A-B are example images acquired by an imaging sensor with a light source having different radiation angles;
Figs. 4A-F are example images acquired by an imaging sensor with a light source configured to emit light with different angles of incidence;
Fig. 5 is a schematic diagram of a device according to an example embodiment; and
Fig. 6 is a flow chart illustrating a method of operating a device according to an embodiment.

### DETAILED DESCRIPTION OF EMBODIMENTS

As noted above, there is provided an improved device for imaging skin, which overcomes existing problems.

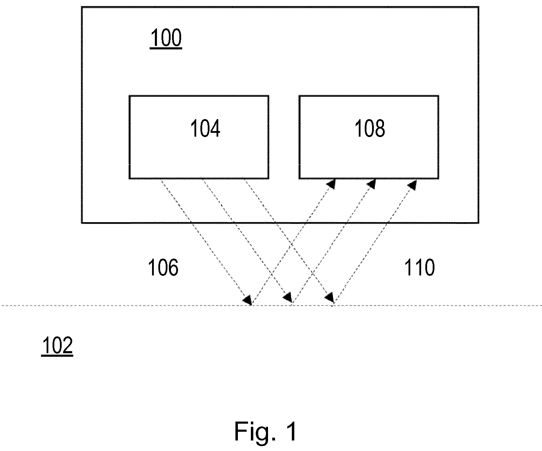

Fig. 1 illustrates a device 100 for imaging skin 102 according to an embodiment. As illustrated in Fig. 1, the device 100 comprises a light source 104. The light source 104 is configured to emit light 106 to illuminate the skin 102. In some embodiments, the light source 104 can, for example, comprise a semiconductor light source such as a light emitting diode (LED), a resonant cavity light emitting diode (RCLED), a vertical cavity laser diode (VCSELs), an edge emitting laser, or any other semiconductor light source. Alternatively or in addition, in some embodiments, the light source 104 can, for example, comprise an organic light source such as a passive-matrix (PMOLED), an active-matrix (AMOLED), or any other organic light source. Alternatively or in addition, in some embodiments, the light source 104 can, for example, comprise a solid state light source. However, although examples for the type of light source 104 have been provided, it will be understood that other types of light source may be used.

As also illustrated in Fig. 1, the device 100 further comprises an imaging sensor 108. The imaging sensor 108 is configured to detect light 110 reflected from the skin 102 and convert the detected light 110 into an image of the skin 102. In some embodiments, the imaging sensor 108 described herein may be, for example, a camera sensor. In some embodiments, the imaging sensor 108 may comprise a focusing lens. Alternatively or in addition, in some embodiments, the imaging sensor 108 may comprise an aperture. In some embodiments, the aperture of the imaging sensor 108 may have a stop size in a range from 0.2 mm to 0.6 mm. Although examples have been provided for the imaging sensor 108, it will be understood that any other type of imaging sensor is also possible.

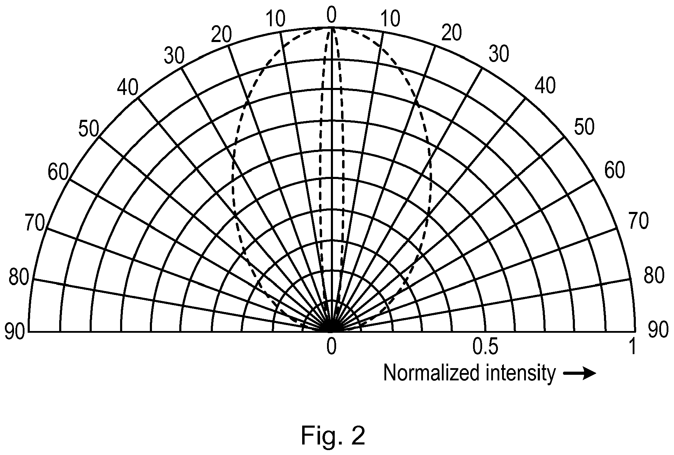

Fig. 2 illustrates a radiation angle range for the light source 104 of the device 100 described herein. The radiation angle referred to herein is the angle at which an intensity (e.g. a luminous intensity) of the light emitted by the light source 104 is half that (i.e. 50%) of a maximum (e.g. peak) intensity of the light emitted by the light source 104. A radiation angle is, for example, a measure of a dispersion (or radiation pattern) of light emitted by a light source. A radiation pattern of a light source viewed on-axis in a narrower viewing angle has a higher intensity than a radiation pattern of a light source viewed on-axis in a wider viewing angle.

As illustrated in Fig. 2, the light source 104 of the device 100 described herein has a radiation angle in a range from 5 to 40 degrees. In some embodiments, for example, the light source 104 of the device 100 can have a radiation angle in a range from 10 to 35 degrees, for example in a range from 15 to 30 degrees, for example in a range from 20 to 25 degrees. In some embodiments, for example, the light source 104 of the device 100 can have a radiation angle in a range from 10 to 30 degrees, for example in a range from 10 to 25 degrees. For example, in some embodiments, the light source 104 of the device 100 can have a radiation angle selected from 10 degrees, 11 degrees, 12 degrees, 13 degrees, 14 degrees, 15 degrees, 16 degrees, 17 degrees, 18 degrees, 19 degrees, 20 degrees, 21 degrees, 22 degrees, 23 degrees, 24 degrees, and 25 degrees, or any non-integer radiation angle between the mentioned values, or any other radiation angle that is in a range from 5 to 40 degrees. Although some examples have been provided for the radiation angle range and the radiation angle for the light source 104 of the device 100, it will be understood that the light source 104 of the device 100 may have any radiation angle in the range from 5 to 40 degrees.

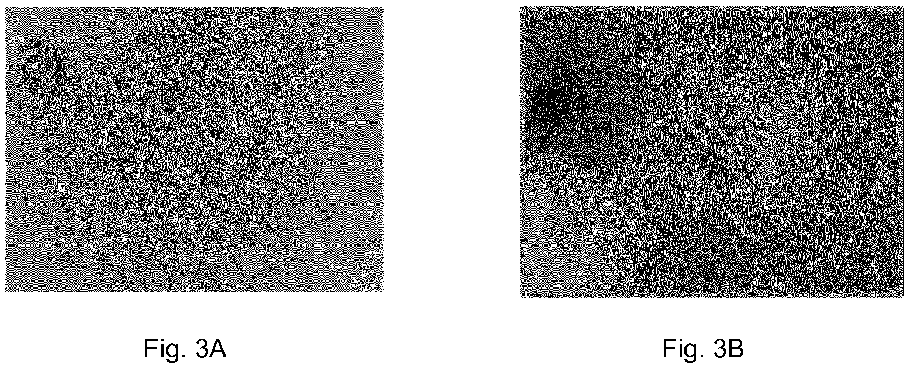

With the light source 104 of the device 100 having the above-described radiation angle, the image of the skin 102 that is acquired from the imaging sensor 108 of the device 100 has an improved resolution. In this way, features of the skin (such as skin texture, fine lines and wrinkles, skin spots, impurities, irregularities, abnormalities, or any other features of the skin) can be better visualized. This is illustrated in Figs. 3A and 3B.

Fig. 3A is an example of an image acquired by an imaging sensor of a device when a light source of the device has a radiation angle of 120 degrees, which falls outside the radiation angle range for the light source 104 of the device 100 described herein. When a light source having a large radiation angle (e.g. any angle above 40 degrees, such as 120 degrees) is used to illuminate an irregular surface such as textured skin, the rays of light emitted by the light source have a broad range of angle of incidence on the surface in the case of diffuse illumination and are therefore less sensitive to any irregularities on the surface. As such, the irregularities are difficult to visualize in an image acquired from an imaging sensor when skin is illuminated with such a light source. By achieving a broad range of angle of incidence for illuminating a textured surface with a large radiation angle (e.g. any angle above 40 degrees, such as 120 degrees), the probability of detecting light reflected from the skin is enhanced, but the sensitivity of the reflected light to any irregularities on the surface of the skin is reduced, which results in an image of poor contrast.

Fig. 3B is an example of an image acquired by an imaging sensor 108 of the device 100 described herein when the light source 104 of the device 100 has a radiation angle of 25 degrees. When the light source 104 of the device 100 described herein having a radiation angle in the range from 5 to 40 degrees is used to illuminate an irregular surface (such as textured skin), the rays of light emitted by the light source have a narrow range of angle of incidence on the surface and are therefore more sensitive to any irregularities on the surface. As such, the irregularities are easier to visualize in an image acquired by the imaging sensor 108 of the device 100 described herein when the skin is illuminated with such a light source 104. Thus, as illustrated in Figs. 3A and 3B, the image acquired with the light source 104 having a radiation angle of 25 degrees has a higher resolution than the image acquired with the light source having a radiation angle of 120 degrees.

In some embodiments, the light source 104 of the device 100 described herein may be configured to emit light 106 with an angle of incidence (AOI) in a particular range. The angle of incidence referred to herein is the angle that the light 106 emitted from the light source 104 (that is, the light that is incident on the skin 102 or that illuminates the skin 102) makes with the normal at a point of incidence on the skin 102.

In some embodiments, the light source 104 of the device 100 may be configured to emit light 106 with an angle of incidence in a range from 5 to 50 degrees. In some embodiments, for example, the light source 104 can be configured to emit light 106 with an angle of incidence in a range from 10 to 45 degrees, for example in a range from 15 to 40 degrees, for example in a range from 20 to 35 degrees, for example in a range from 25 to 30 degrees. In some embodiments, for example, the light source 104 can be configured to emit light 106 with an angle of incidence in a range from 10 to 30 degrees, for example in a range from 10 to 25 degrees. For example, in some embodiments, the light source 104 can be configured to emit light 106 with an angle of incidence selected from 10 degrees, 11 degrees, 12 degrees, 13 degrees, 14 degrees, 15 degrees, 16 degrees, 17 degrees, 18 degrees, 19 degrees, 20 degrees, 21 degrees, 22 degrees, 23 degrees, 24 degrees, and 25 degrees, or any non-integer angle of incidence between the mentioned values, or any other angle of incidence that is in a range from 5 to 50 degrees. Although some examples have been provided for the angle of incidence range and the angle of incidence for the light source 104 of the device 100, it will be understood that the light source 104 may be configured to emit light 106 with any angle of incidence in the range from 5 to 50 degrees.

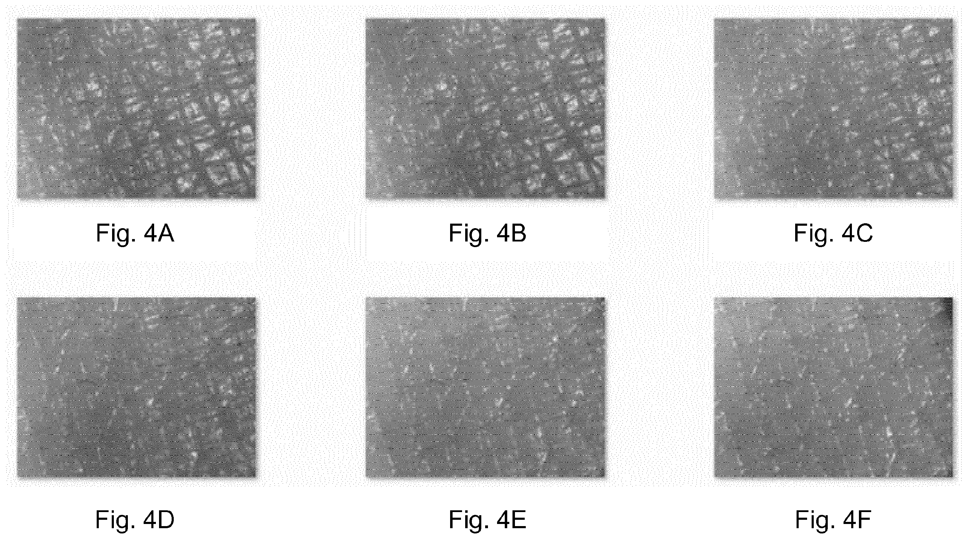

Fig. 4A is an example of an image acquired by the imaging sensor 108 of the device 100 when the light source 104 of the device 100 is configured to emit light 106 with an angle of incidence of 15 degrees. Fig. 4B is an example of an image acquired by the imaging sensor 108 of the device 100 when the light source 104 of the device 100 is configured to emit light 106 with an angle of incidence of 20 degrees. Fig. 4C is an example of an image acquired by the imaging sensor 108 of the device 100 when the light source 104 of the device 100 is configured to emit light 106 with an angle of incidence of 30 degrees. Fig. 4D is an example of an image acquired by the imaging sensor 108 of the device 100 when the light source 104 of the device 100 is configured to emit light 106 with an angle of incidence of 40 degrees. Fig. 4E is an example of an image acquired by the imaging sensor 108 of the device 100 when the light source 104 of the device 100 is configured to emit light 106 with an angle of incidence of 50 degrees. Fig. 4F is an example of an image acquired by an imaging sensor of a device when a light source of the device is configured to emit light with an angle of incidence of 60 degrees.

As illustrated in Figs. 4A-F, the images acquired by the imaging sensor 108 of the device 100 when the light source 104 of the device 100 is configured to emit light 106 with an angle of incidence in the range from 5 to 50 degrees (as illustrated in Figs. 4A-E) have a higher resolution than the image acquired by an imaging sensor of a device when a light source of the device is configured to emit light outside this range, specifically with an angle of incidence of 60 degrees (as illustrated in Fig. 4F). Thus, an angle of incidence in the range from 5 to 50 degrees is optimal for resolving features of the skin (such as skin texture, fine lines and wrinkles, skin spots, impurities, irregularities, abnormalities, or any other features of the skin), whereas at a larger angle of incidence such features of the skin may not be visible in acquired images.

In some embodiments, the light source 104 may be configured to emit light 106 to uniformly (or homogeneously) illuminate the skin 102. For example, in some embodiments, the light source 104 may be configured to emit light 106 to provide a uniform (or homogenous) light intensity distribution toward the skin 102. More specifically, for example, the light source 104 may be configured to emit light 106 to uniformly illuminate the skin 102 in the field of view of the imaging sensor 108 or to provide a uniform intensity distribution of light toward the skin 102 in the field of view of the imaging sensor 108.

In some embodiments, the light source 104 may comprise a plurality of light emitters suitably arranged to provide uniform illumination to the skin 102. For example, the plurality of light emitters may be provided in a ring or tiled arrangement to provide uniform illumination to the skin 102. Alternatively or in addition, the plurality of light emitters may be tilted to provide uniform illumination to the skin 102. While it has been described that the light source 104 may be configured to emit light 106 to uniformly illuminate the skin 102, it will be appreciated that in some embodiments, the light source 104 may be configured to emit light 106 to substantially uniformly illuminate the skin 102 or to emit light 106 to (e.g. substantially) uniformly illuminate an area of skin that is of a predefined size. Although some examples have been provided for the manner in which the light source 104 may be configured to emit light 106 to uniformly illuminate the skin 102, a person skilled in the art will also be aware of other examples. Thus, according to some embodiments, the illumination conditions can prevent non-linear effects or non-uniformities for intensity being present in the field of view.

Thus, as described earlier, the light source 104 of the device 100 described herein has a radiation angle in a range from 5 to 40 degrees. In some embodiments, the light source 104 of the device 100 may also be configured to emit light 106 with an angle of incidence in a range from 5 to 50 degrees. Alternatively or in addition, according to some embodiments, the light source 104 may be configured or further configured to emit light 106 to uniformly illuminate the skin 102. In this way, images acquired by the imaging sensor 108 of the device 100 have an improved resolution, such that it is possible to optimally resolve features of the skin (such as skin texture, fine lines and wrinkles, skin spots, impurities, irregularities, abnormalities, or any other features of the skin) in the acquired images. There is thus described herein a device 100 that provides optimal illumination conditions for imaging skin.

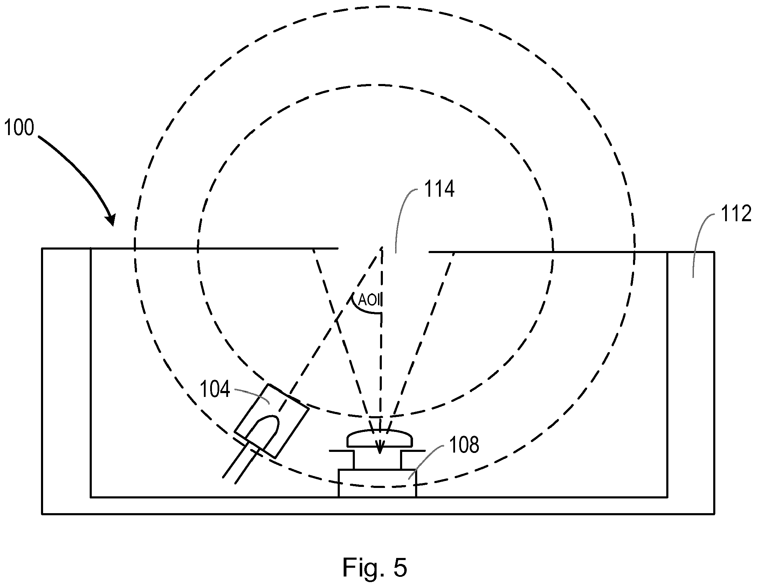

Fig. 5 illustrates the device 100 for imaging skin 102 according to an example embodiment. The device 100 according to this illustrated example embodiment is as described earlier with reference to Fig. 1. In particular, the device 100 comprises a light source 104 configured to emit light 106 to illuminate the skin 102 and an imaging sensor 108 configured to detect light 110 reflected from the skin 102 and convert the detected light 110 into an image of the skin 102. As described earlier with reference to Fig. 2, the light source 104 of the device 100 has a radiation angle in a range from 5 to 40 degrees. In some embodiments, as described earlier, the light source 104 of the device 100 may be configured to emit light 106 with an angle of incidence in a range from 5 to 50 degrees. In some embodiments, as also described earlier, the light source 104 may be configured to emit light 106 to uniformly illuminate the skin 102.

In the example embodiment illustrated in Fig. 5, the device 100 further comprises a housing 112 configured to house the light source 104 and the imaging sensor 108. The housing 112 comprises an aperture 114 through which the light source 104 is configured to emit light 106 and the imaging sensor 108 is configured to detect light 110 reflected from the skin 102. In some embodiments, the aperture 114 can be a numerical aperture. In some of these embodiments, the numerical aperture may have a value in a range from 0.005 to 0.3. In some embodiments, for example, the numerical aperture may have a value in a range from 0.01 to 0.275, for example in a range from 0.015 to 0.25, for example in a range from 0.02 to 0.225, for example in a range from 0.025 to 0.2, for example in a range from 0.03 to 0.175, for example in a range from 0.035 to 0.15, for example in a range from 0.04 to 0.125, for example in a range from 0.045 to 0.1, for example in a range from 0.05 to 0.075. Although some examples have been provided for the numerical aperture value range and the numerical aperture value of the housing 112 of the device 100, it will be understood that the numerical aperture of the housing 112 of the device 100 may be any value in the range from 0.005 to 0.3.

In some embodiments, the light source 104 described herein may be configured to emit unpolarized light. In some of these embodiments, the device 100 may further comprise a polarizer (not illustrated). The polarizer can be configured to polarize the light 110 reflected from the skin 102. Thus, the polarizer may be positioned such that light 110 reflected from the skin 102 reaches the polarizer before the imaging sensor 108. For example, the polarizer can be positioned before (e.g. in front of) the imaging sensor 108. In embodiments where the device 100 comprises a polarizer, the imaging sensor 108 can be configured to detect the polarized light 110 reflected from the skin 102. Alternatively, in other embodiments, the light source 104 described herein may be configured to emit polarized light. In these embodiments, the imaging sensor 108 can be configured to detect the polarized light 110 reflected from the skin 102.

In some embodiments, the light source 104 described herein may comprise a single light emitter. In other embodiments, the light source 104 described herein may comprise a plurality of light emitters. For example, according to some embodiments, the light source 104 described herein may comprise at least two light emitters, at least three light emitters, at least four light emitters, at least five light emitters, at least six light emitters, or any other number of light emitters. In embodiments where the light source 104 comprises a plurality of light emitters, the plurality of light emitters may be configured together as a single lighting module. In some embodiments, the light source 104 (and thus the one or more light emitters) may be provided (or mounted) on a substrate, such as a semiconductor substrate or a printed circuit board (PCB). Thus, according to some embodiments, the light source 104 may be a chip-on-board (COB) light source. In some embodiments where the light source 104 comprises a plurality of light emitters, the plurality of light emitters may be provided (or mounted) on the same substrate.

The plurality of light emitters may comprise any type of light emitter or any combination of types of light emitter, such as any of the types provided earlier in respect of the light source 104 of the device 100. For example, the plurality of light emitters may comprise any one or more of a semiconductor light emitter (such as a light emitting diode LED, a resonant cavity light emitting diode RCLED, a vertical cavity laser diode VCSEL, an edge emitting laser, or any other semiconductor light emitter, or any combination of semiconductor light emitter), an organic light emitter (such as a passive-matrix PMOLED, an active-matrix AMOLED, or any other organic light emitter, or any combination of organic light emitters), a solid state light emitter, or any other light emitter, or any combination of light emitters.

In some embodiments, the light source 104 described herein may be configured to emit visible light, such as white light. In some embodiments where the light source 104 is configured to emit visible light, the visible light may have a wavelength in a range from 389 nm to 780 nm. In some embodiments where the light source 104 is configured to emit white light, the white light may have a correlated color temperature (CCT) in a range from 2000 K to 20000 K, for example in a range from 2700 K to 20000 K, for example in a range from 2700 K to 6500 K. Alternatively or in addition to visible light, in some embodiments, the light source 104 described herein may be configured to emit infrared (IR) light, such as near infrared (NIR) light. In some embodiments where the light source 104 is configured to emit infrared light, the infrared light may have a wavelength in a range from 750 nm to 3000 nm. Although some examples have been provided for the type of light source 104, it will be appreciated that the device 100 may alternatively or in addition comprise any other type of light source or light sources suitable to illuminate the skin 102.

In some embodiments, a distance between the light source 104 and the imaging sensor 108 of the device 100 described herein may be greater than 5 millimeters (mm). In some embodiments, a distance between the light source 104 and the imaging sensor 108 of the device 100 described herein may be in a range from 6 mm to 14 mm, for example in a range from 7 mm to 13 mm, for example in a range from 8 mm to 12 mm, for example in a range from 9 mm to 11 mm. For example, in some embodiments, the distance between the light source 104 and the imaging sensor 108 may be a distance selected from 6 mm, 7 mm, 8 mm, 9 mm, 10 mm, 11 mm, 12 mm, 13 mm and 14 mm, or any non-integer distance between the mentioned values, or any other distance greater than 5 millimeters. In any of embodiments described herein, the imaging sensor 108 of the device 100 described herein may have a particular depth of focus (DOF). For example, in some embodiments, the imaging sensor 108 may have a depth of focus (DOF) that is greater than 2 mm.

Although not illustrated, there is also provided a system for measuring skin texture. The system comprises a device 100 according to any of the embodiments described herein. The system also comprises a processor. The processor is configured to acquire the image of the skin 102 from the imaging sensor 108 and process the acquired image to measure the skin texture. By implementing the device 100 according to any of the embodiments described herein in the system, optimal illumination conditions (or settings) can be provided for resolving skin texture. Thus, an improved system for measuring skin texture is provided.

In some embodiments, the processor may be configured to detect a color contrast in the acquired image and measure the skin texture by determining (or deriving) a two-dimensional texture parameter from the detected color contrast in the acquired image. In embodiments where the device 100 comprises a housing 112 configured to house the light source 104 and the imaging sensor 108 and the housing 112 comprises an aperture 114 (such as in the example embodiment illustrated in Fig. 5), the color contrast in the acquired image.

In some embodiments, the device 100 itself may comprise the processor. In other embodiments, the processor may be external to (i.e. separate to or remote from) the device 100. For example, in some embodiments, the processor may be a separate entity or part of another device.

The processor can be implemented in numerous ways, with software and/or hardware, to perform the various functions described herein. In particular implementations, the processor can comprise a plurality of software and/or hardware modules, each configured to perform, or that are for performing, individual or multiple steps of the method described herein. The processor may comprise one or more processors (such as one or more microprocessors, one or more multi-core processors and/or one or more digital signal processors (DSPs)), one or more processing units, and/or one or more controllers (such as one or more **microcontrollers**) that may be configured or programmed (e.g. using software or computer program code) to perform the various functions described herein. The processor may be implemented as a combination of dedicated hardware (e.g. amplifiers, pre-amplifiers, analog-to-digital convertors (ADCs) and/or digital-to-analog convertors (DACs)) to perform some functions and a processor (e.g. one or more programmed microprocessors, controllers, DSPs and associated circuitry) to perform other functions.

Although the device 100 has been described herein with reference to Figs. 1 and 5, it will be appreciated that Figs. 1 and 5 only show the components required to illustrate certain embodiments and, in a practical implementation, the device 100 may comprise other components or additional components to those shown.

For example, although not illustrated in Figs. 1 or 5, the device 100 may comprise a communications interface (or communications circuitry). The communications interface can be for enabling the device 100 or components of the device 100 to communicate with and/or connect to one or more other devices, components, interfaces, memories, or sensors (such as any of those described herein). For example, the communications interface can be for enabling the imaging sensor 108 of the device 100 to communicate with and/or connect to the processor described earlier for image processing. The communications interface may be configured to communicate wirelessly, via a wired connection, or via any other communication (or data transfer) mechanism. In some wireless embodiments, the communications interface may, for example, use radio frequency (RF), Bluetooth, or any other wireless communication technologies, for communications.

Although also not illustrated in Figs. 1 or 5, the device 100 may comprise a memory, or may be configured to communicate with and/or connect to a memory external to (i.e. separate to or remote from) the device 100. The memory may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). The memory can be configured to store program code that can be executed by a processor to cause the device 100 to operate in the manner described herein. Alternatively or in addition, the memory can be configured to store information acquired by the device 100. For example, in some embodiments, the memory may be configured to store an image of skin acquired by the imaging sensor 108 of the device 100, a skin texture measured by the processor of the system, or any other information, or any combination of information resulting from the method described herein.

Although also not illustrated in Figs. 1 or 5, the device 100 may comprise a user interface, or may be configured to communicate with and/or connect to a user interface external to (i.e. separate to or remote from) the device 100. The user interface can be configured to render (or output, display, or provide) information resulting from the method described herein. For example, in some embodiments, the user interface may be configured to render (or output, display, or provide) an image of skin acquired by the imaging sensor 108, a skin texture measured by the processor, or any other information, or any combination of information resulting from the method described herein. Alternatively or in addition, the user interface can be configured to receive a user input. For example, the user interface may allow a user to manually enter information or instructions, interact with and/or control the device 100. Thus, the user interface may be any user interface that enables the rendering (or outputting, displaying, or providing) of information and, alternatively or in addition, enables a user to provide a user input.

For example, the user interface may comprise one or more switches, one or more buttons, a keypad, a keyboard, a mouse, a touch screen or an application (for example, on a smart device such as a tablet, a smartphone, or any other smart device), a display or display screen, a graphical user interface (GUI) or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render information may be the same user interface as that which enables the user to provide a user input.

Although also not illustrated in Figs. 1 or 5, the device 100 may comprise a battery or other power supply for powering the device 100 or means for connecting the device 100 to a mains power supply, or any other component, or any combination of components.

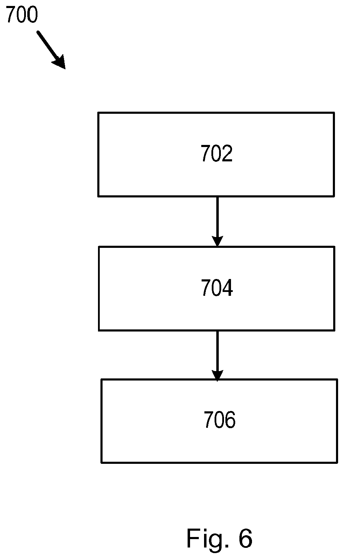

Fig. 6 illustrates a method 700 of operating the device 100 described herein to image skin 102 according to an embodiment. In some embodiments, the method 700 may generally be performed by or under the control of a processor, such as the processor mentioned earlier. At block 702, light is emitted from the light source 104 of the device 100 to illuminate the skin 102. At block 704, light reflected from the skin is detected by the imaging sensor 108 of the device 100. At block 706, the detected light is converted by the imaging sensor 108 into an image of the skin. Although not illustrated in Fig. 6, according to some embodiments, the method 700 may further comprise a processor processing the acquired image to measure skin texture, as described earlier.

There is also provided a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an improved device 100 for imaging skin 102, a method 700 of operating the device 100 to image skin 102, an improved system for measuring skin texture, a method of operating the system to measure skin texture, and a computer program product. According to these aspects, images of improved resolution (and thus improved quality) can be acquired. The resolution of the images achieved by the device 100 described herein is optimum for adequately resolving features of the skin (such as skin texture, fine lines and wrinkles, skin spots, impurities, irregularities, abnormalities, or any other features of the skin). For example, it can be possible to achieve a resolution of more than 20 lines per millimeter (lnp/mm), for example a resolution of more than 25 lnp/mm. In effect, an optimal parameter space for resolving skin texture is provided. This is possible through the use of a small radiation angle light source 104 (e.g. with maximum intensity distribution on the optical axis) and optionally also with one or more of the light source 104 having a small angle of incidence and being configured to provide a uniform light intensity distribution (e.g. in a narrow range of angles in the field of view). Thus, according to the aspects and embodiments described herein, images acquired by the imaging sensor 108 of the device 100 can be used to provide more accurate skin monitoring and to more reliably detect skin conditions.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A device (100) for imaging skin (102), the device (100) comprising:
a light source (104) configured to emit light (106) to illuminate the skin (102), the light source (104) having a radiation angle in a range from 5 to 40 degrees, wherein the radiation angle is the angle at which an intensity of the light emitted by the light source (104) is half that of a maximum intensity of the light (106) emitted by the light source (104); and
an imaging sensor (108) configured to detect light (110) reflected from the skin (102) and convert the detected light (110) into an image of the skin (102).

2. A device (100) as claimed in claim 1, wherein the light source (104) has a radiation angle in a range from 10 to 30 degrees.

3. A device (100) as claimed in claim 2, wherein the light source (104) has a radiation angle in a range from 10 to 25 degrees.

4. A device (100) as claimed in any one of the preceding claims, wherein the light source (104) is configured to emit light (106) with an angle of incidence in a range from 5 to 50 degrees.

5. A device (100) as claimed in claim 4, wherein the light source (104) is configured to emit light (106) with an angle of incidence in a range from 10 to 30 degrees.

6. A device (100) as claimed in claim 5, wherein the light source (104) is configured to emit light (106) with an angle of incidence in a range from 10 to 25 degrees.

7. A device (100) as claimed in any one of the preceding claims, wherein the light source (104) is configured to emit light (106) to uniformly illuminate the skin.

8. A device (100) as claimed in any one of the preceding claims, wherein the light source (104) comprises a plurality of light emitters.

9. A device (100) as claimed in claim 8, wherein the light source (104) comprises at least three light emitters.

10. A device (100) as claimed in any one of the preceding claims, wherein the device (100) further comprises:
a housing (112) configured to house the light source (104) and the imaging sensor (108),
wherein the housing (112) comprises an aperture (114) through which the light source (104) is configured to emit light (106) and the imaging sensor (108) is configured to detect light (110) reflected from the skin (102).

11. A device (102) as claimed in claim 10, wherein the aperture (114) is a numerical aperture having a value in a range from 0.005 to 0.3.

12. A device (100) as claimed in any one of the preceding claims, wherein a distance between the light source (104) and the imaging sensor (106) is greater than 5 millimeters.

13. A system for measuring skin texture, the system comprising:
a device (100) as claimed in any one of the preceding claims; and
a processor configured to acquire the image of the skin (102) from the imaging sensor (108) and process the acquired image to measure the skin texture.

14. A method (700) of operating a device (100) to image skin (102), wherein the device (100) comprises a light source (104) and an imaging sensor (108), and the method (700) comprises:
emitting (702) light (106) from the light source (104) to illuminate the skin (102), wherein the light source (104) has a radiation angle in a range from 5 to 40 degrees, wherein the radiation angle is the angle at which an intensity of the light (106) emitted by the light source (104) is half that of a maximum intensity of the light (106) emitted by the light source (104);
detecting (704), by the imaging sensor (108), light (110) reflected from the skin (102); and
converting (706), by the imaging sensor (108), the detected light (110) into an image of the skin (102).

15. A computer program product comprising a computer readable medium, the computer readable medium having computer readable code embodied therein, the computer readable code being configured such that, on execution by a suitable computer or processor, the computer or processor is caused to perform the method as claimed in claim 14.
